# FASCICULE DE BREVET EUROPEEN

(11) **EP 0 381 964 B1**
(45) Date de publication et mention de la délivrance du brevet: **17.03.1993**
(21) Numéro de dépôt: 90100997.7
(22) Date de dépôt: 18.01.1990
(51) Int. Cl.: C07C 13/00, C07C 1/24

(54) **Procédé pour la préparation de composés cycliques polyinsaturés**
Verfahren zur Herstellung von mehrfach ungesättigten cyclischen Verbindungen
Process for the preparation of polyunsaturated cyclic compounds

(30) Priorité: 09.02.1989 CH 442/89
(43) Date de publication de la demande: 16.08.1990
(73) Titulaire: FIRMENICH SA, CH-1211 Genève 8 (CH)
(72) Inventeur: Mahaim, Cyril Jacques, CH-1112 Echichens (CH)
(74) Mandataire: Salvadori, Giuseppe

(56) Documents cités:
- US-A- 4 568 782
- US-A- 4 677 238

## Description

L'invention se rapporte au domaine de la synthèse organique. En particulier, elle concerne un procédé pour la préparation de composés cycliques polyinsaturés, utiles à titre de produits intermédiaires dans la synthèse de cétones cycliques et macrocycliques odorantes, la muscone en particulier.

La muscone est une cétone macrocyclique fort appréciée dans l'art de la parfumerie pour son odeur musquée et nombreuses sont les méthodes décrites à ce jour pour sa préparation. Les méthodes connues ne s'appliquent toutefois qu'avec difficulté à l'exploitation industrielle à grande échelle, soit à cause de leur complexité, soit en raison du faible rendement de certaines étapes critiques. L'une des méthodes connues fait appel aux composés de formule 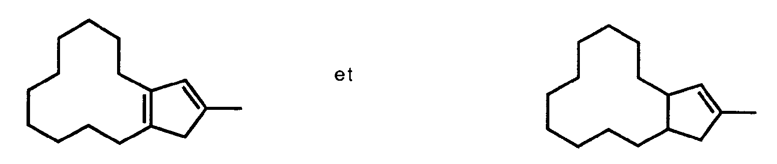
[voir DE-OS 29 16 418, publiée le 6.11.1980], lesquels composés sont ensuite soumis à une hydrogénation, respectivement une isomérisation acide, pour fournir un composé de formule 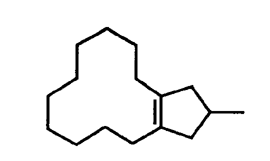
qui, par ozonisation suivie de réduction, fournit la muscone désirée.

Les composés utilisés comme produits de départ dans le procédé cité ont été obtenus par une voie de synthèse à plusieurs étapes telle qu'illustrée ci-dessous :

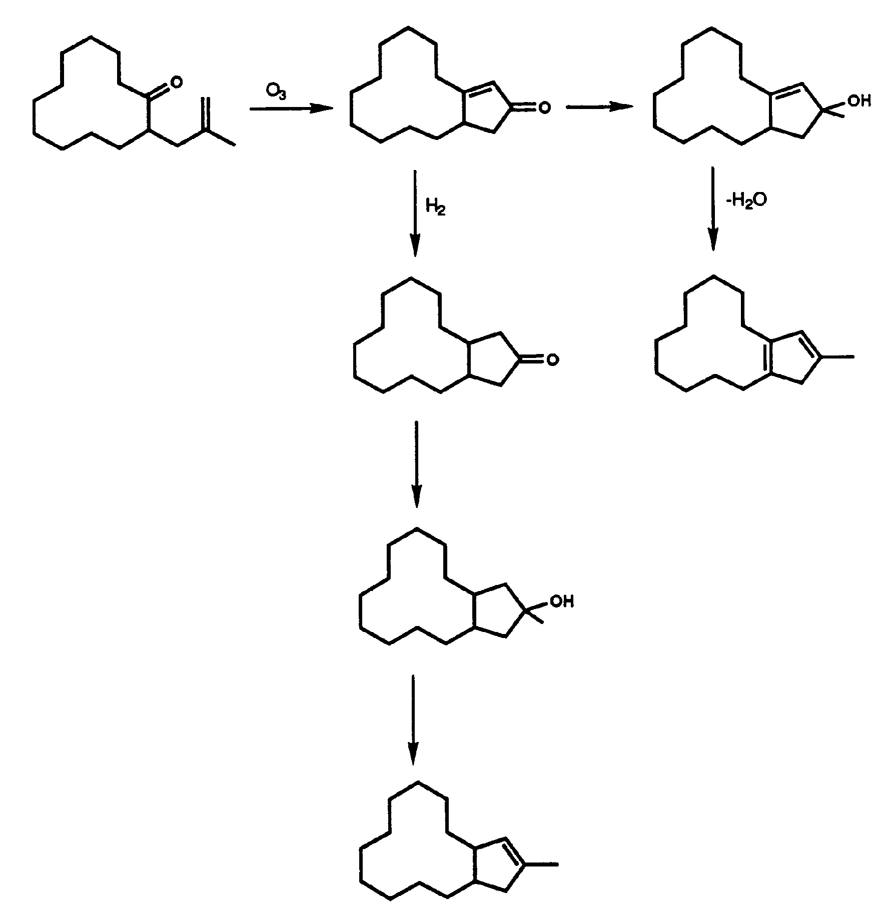

Une autre voie de synthèse a recours au composé de formule 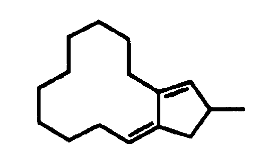
[voir brevet européen N° 37116]. Ce composé a été préparé par un procédé qui consiste en l'addition en milieu alcalin du chlorure de méthallyle à la cyclododécanone suivie de l'addition au produit ainsi obtenu de thiophénol, oxydation au moyen d'un peroxyde organique et traitement avec une base forte de la sulfone résultante.

L'invention a le mérite de proposer un procédé nouveau, original, permettant d'obtenir des composés cycliques polyinsaturés de formule 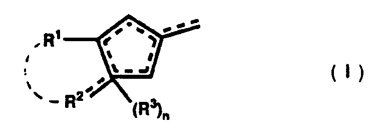
possédant deux doubles liaisons conjugées dans les positions endo- et exo- du cycle pentagonal indiquées par les lignes pointillées, et dans laquelle les symboles R¹ et R², pris séparément, représentent respectivement a) un radical alkyle linéaire ou ramifié de C₁ à C₄ et b) un atome d'hydrogène ou un radical méthyle, ou, pris ensemble avec les atomes de carbone auxquels ils sont liés, forment un cycle polyméthylénique tel qu'indiqué par la ligne pointillée contenant de 5 à 12 atomes de carbone, et le symbole R³ représente un atome d'hydrogène ou un radical méthyle, n étant égal à 0 ou 1.

Le procédé selon l'invention consiste à soumettre une énone de formule 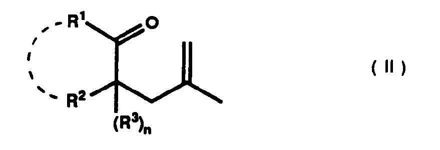
dans laquelle la ligne pointillée et les symboles n, R¹, R², R³ ont le sens indiqué ci-dessus, a une cyclisation, en phase vapeur, au moyen d'un traitement thermique en présence d'un catalyseur de cyclisation constitué par un oxyde d'aluminium, de silicium, de titane ou de zirconium.

Sans vouloir préjuger de l'exactitude de notre hypothèse, il est vraisemblable que la réaction procède selon le schéma d'une réaction "ène" intramoléculaire tel qu'illustré ci-dessous :

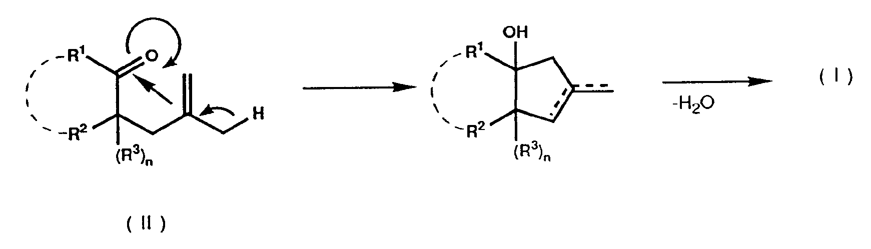

Le procédé de l'invention s'effectue de préférence à une température comprise entre 200 et 400°C, quoique les rendements les meilleurs ont été obtenus dans une gamme comprise entre 280 et 320°C. A des températures inférieures, nous avons observé une augmentation de la formation de produits secondaires sous forme d'éthers cycliques de formule 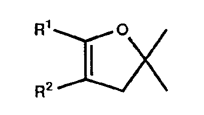
tandis qu'au-delà de la limite supérieure indiquée, nous avons observé une diminution du rendement, cela étant probablement dû à des réactions de craquage.

La réaction s'effectue de préférence à pression réduite. La valeur de pression choisie est dictée par un compromis entre le fait d'obtenir un débit suffisant et constant des vapeurs de l'énone de départ et la nécessité d'avoir un temps de contact suffisant entre les vapeurs formées et le catalyseur pour obtenir une conversion satisfaisante.

Nous avons pu déterminer qu'une pression comprise entre 1 x 10³ et 13 x 10³ Pa était parfaitement adaptée en la circonstance. Toutefois, la réaction peut s'effectuer à des pressions supérieures aux limites indiquées, par exemple à pression atmosphérique.

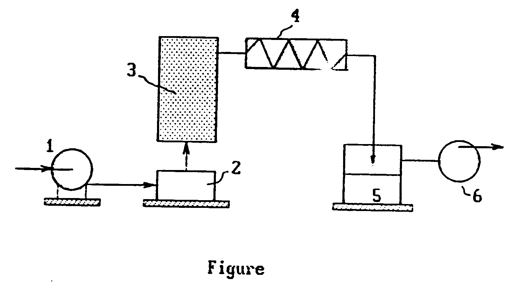

Une forme d'exécution particulière de l'appareillage nécessaire à la mise en oeuvre du procédé de l'invention est décrite à titre d'exemple dans le dessin annexé.

La figure en représente un schéma. L'énone de départ est introduite dans un four de préchauffage 2 à l'aide d'une pompe doseuse 1 et les vapeurs ainsi formées sont forcées à travers le catalyseur déposé dans une colonne chauffée 3. Les vapeurs du produit résultant sont ensuite condensées au moyen d'un système de refroidissement et trappe 4 et recueillies dans un récipient 5. Le niveau de pression désiré est atteint au moyen d'une pompe d'aspiration 6.

Comme indiqué ci-dessus, les vapeurs de l'énone de départ entrent en contact avec le catalyseur de cyclisation contenu dans une colonne préchauffée à la température choisie de réaction. Le débit des vapeurs de ladite énone dans la colonne de catalyseur est maintenu à une valeur constante, d'une part par l'aspiration exercée sur la colonne et d'autre part par une introduction régulière de l'énone liquide au moyen d'une pompe doseuse.

Le produit désiré, en mélange avec l'énone de départ n'ayant pas réagi, est ensuite recueilli par condensation des vapeurs obtenues à la sortie de la colonne.

Parmi les catalyseurs aptes à promouvoir la cyclisation du produit de départ, il convient de mentionner l'alumine, la silice, le ZrO₂, le ZnO et le TiO₂. Il s'agit sans exception de produits aisément disponibles sur le marché. L'alumine représente le réactif préférentiel.

La proportion entre la quantité de catalyseur et celle du produit de départ n'est pas critique, elle peut donc varier dans une gamme de valeurs assez étendue. Des proportions de l'ordre de 1 : 52 (alumine : énone) ont été utilisées sans qu'on ait remarqué une baisse notable de rendement. La valeur du débit des vapeurs d'énone de départ qui circulent à travers la colonne peut également varier sans que cela ait une influence majeure sur les rendements de cyclisation. Un débit de 0,2 - 1 ml d'énone par ml de volume utile et par heure, de préférence de 0,4 à 0,6, est parfaitement adapté en la circonstance. On définit comme volume utile le volume occupé par le catalyseur dans la colonne le contenant.

Les exemples suivants illustrent l'invention d'une manière plus détaillée (les températures sont indiquées en degrés centigrades et les abréviations ont le sens usuel dans l'art).

L'appareillage utilisé est schématiquement illustré à la figure donnée en annexe.

### Exemple 1

### Préparation de 14-méthyl-bicyclo[10.3.0]pentadeca-1(12),13-diène

Le volume utile de la colonne de catalyseur employée était de 50 ml et la charge d'alumine de 22,5 g (Al₂O₃, Harshaw AI 3996 R).
1300 g de 2-(2-méthyl-2-propényl)-cyclododécanone ou méthallylcyclododécanone ont été introduits dans un évaporateur à l'aide d'une pompe doseuse réglée de façon à ce que le débit obtenu soit de 10 ml/h. L'évaporateur a été maintenu à une température de 260° et la pression appliquée de 1,1 x 10⁴ Pa.
La colonne chargée d'alumine a été maintenue à une température constante d'environ 260°.
Les vapeurs résultantes ont été ensuite condensées à l'aide d'un réfrigérant et le produit désiré a été séparé par décantation (rend. 94%).
La même opération a été effectuée en utilisant comme catalyseur l'un des oxydes métalliques suivants :
gamma-Al₂O₃ Akzo 001-3 P
gamma-Al₂O₃ Akzo 001-1,5 E
gamma-Al₂O₃ BASF D10-10
gamma-Al₂O₃ BASF D10-20
gamma-Al₂O₃ Rhône-Poulenc SAS 350
SiO₂ BASF D11-10
gamma-Al₂O₃/H₃PO₄ Harshaw
ZrO₂ Harshaw
ZnO BASF
TiO₂ Harshaw
La réaction a été également conduite à 250° et 300°. Les essais ont montré que la cyclisation de la méthallylcyclododécanone s'effectuait sans différences notables dans les limites des conditions choisies.
RMN(¹H, 360MHz, CDCl₃) : 1,00-1,80(m, 16H) ; 2,00(s large, 3H) ; 2,26(t, J=6Hz, 2H); 2,34(t, J=6Hz, 2H); 2,79(s large, 2H); 5,96(s large, 1H) δ ppm.
SM: 218(6, M⁺), 203(1), 189(0), 175(1), 161(4), 147(0), 133(11), 119(24), 107(39), 94(100), 91(40), 79(19), 67(6), 55(8), 41(7).

### Exemple 2

### Préparation de 4,5,6,7-tétrahydro-2-méthyl-1H-indène

Le volume utile de la colonne de catalyseur employée était de 20 ml et la charge d'alumine de 9,0 g (Al₂O₃, Harshaw AI 3996 R). 100 g de 2-(2-méthyl-2-propényl)-cyclohexanone ou méthallylcyclohexanone ont été introduits dans un évaporateur à l'aide d'une pompe doseuse réglée de façon à ce que le débit obtenu soit de 8,5 ml/h. L'évaporateur a été maintenu à une température de 270 à 290° et la pression appliquée était de 1,1 x 10⁴ Pa. On a coalimenté d'eau à un débit de 3 ml/h pour prolonger la vie du catalyseur.
Les vapeurs résultantes ont été ensuite condensées à l'aide d'un réfrigérant et on a obtenu un mélange du produit désiré avec d'autres isomères (rend : 72%).
Le produit désiré, représentant environ 40% dudit mélange, a été séparé par distillation sur colonne Fischer.
P. eb. 65°/1,5 x10³ Pa.
RMN(¹H, 360MHz, CDCl₃) : 1,60-1,80(s large, 4H) ; 2,05(s large, 3H) ; 2,10-2,35(m, 4H) ; 2,75(s large, 2H) ; 5,90(s large, 1H) δ ppm.
SM : 134(28, M⁺), 119(33), 106(38), 105(43), 91(100).

### Exemple 3

### Préparation de 4,5,6,7-tétrahydro-2,3a,7,7-tétraméthyl-3aH-indène

Le volume utile de la colonne de catalyseur employée était de 20 ml et la charge d'alumine de 9,6 g (Al₂O₃, Harshaw Al 3996 R). 38,5 g de 2,2,6-triméthyl-6-(2-méthyl-2-propényl)-cyclohexanone ont été introduits dans un évaporateur à l'aide d'une pompe doseuse réglée de façon à ce que le débit obtenu soit de 8,3 ml/h.
L'évaporateur a été maintenu à une température de 270° et la pression appliquée était de 1,6 x 10³ Pa.
Les vapeurs résultantes ont été ensuite condensées à l'aide d'un réfrigérant et on a obtenu 20,8 g d'un mélange contenant environ 60% du produit désiré, 20% d'énone de départ non réagie et 12% d'un isomère du produit désiré de structure non identifiée.
Le produit désiré a été séparé par distillation sur colonne Fischer.
P. eb. 88-89°/1,6 x 10³ Pa.
RMN(¹H, 360MHz, CDCl₃) : 0,85-1,23(m, 2H) ; 1,13(s, 3H) ; 1,14(s, 3H) ; 1,45-1,95(m, 4H) ; 1,85(s large, 3H) ; 5,74(s large, 1H) ; 5,76(s large, 1H) δ ppm.
SM : 176(5, M⁺), 161(100), 147(2), 133(11), 119(20), 105(13), 91(14), 77(5).

### Exemple 4

### Préparation de 1,3-diméthyl-1,3-cyclopentadiène

Le volume utile de la colonne de catalyseur employée était de 30 ml et la charge d'alumine de 16,1 g (Al₂O₃, Harshaw Al 3996 R). 354 g de 5-méthyl-5-hexén-2-one ont été introduits dans un évaporateur à pression atmosphérique, l'évaporateur ayant été maintenu à une température de 260 à 280°. La température a été élevée pendant le cours de la réaction afin de maintenir la conversion. On a coalimenté d'eau à un débit de 3 ml/h pour le débit de 15 ml/h d'énone de départ.
Les vapeurs résultantes ont été ensuite condensées à l'aide d'un réfrigérant et on a obtenu 271 g d'un mélange contenant environ 80% du produit désiré et des quantités moindres de cétone de départ et d'autres isomères du produit désiré.
Ce dernier a été séparé par distillation à pression atmosphérique.
P. eb. 62°/1 x 10⁵ Pa.
RMN(¹H, 360MHz, CDCl₃) : 1,95(s large, 3H) ; 2,05(s large, 2H) ; 2,85(m, 2H) ; 5,70(s large, 1H) ; 5,00(s large, 1H) δ ppm.
SM : 94(46, M⁺), 93(77), 91(29), 77(56).

### Exemple 5

### Préparation de 1,3.5,5-tétraméthyl-1,3-cyclopentadiène

a) 3,3-diméthyl-5-méthyl-5-hexén-2-one
   93 g de NaOH, 12 g de Nal et 6,8 g de Emkapol 400 (polyéthylèneglycol) ont été chargés dans un ballon à trois cols de 1 l. On a introduit 100 g de méthylisopropylcétone et, à 50°, 290 g de chlorure de méthallyle. On a porté le mélange à reflux pendant 4 heures et ensuite hydrolysé avec 200 ml d'eau. On a lavé à neutralité et distillé le mélange pour obtenir 69,6 g de produit brut (P. eb. 56-65°/5,0 x 10³ Pa). La distillation sur colonne Widmer de ce produit brut a fourni 44,5 g de la cétone désirée.
   P. eb. 57-60°/3 x 10³ Pa.
   RMN(¹H, 360MHz, CDCl₃) : 1,15(s, 6H) ; 1,65(s, 3H) ; 2,15(s, 3H) ; 2,30(s, 2H) ; 4,65(s, 1H) ; 4,80(s, 1H) δ ppm.
   SM : 160(20, M⁺), 125(8), 197(11), 97(64), 83(8), 69(33), 55(100), 43(44).
b) Le volume utile de la colonne de catalyseur employée était de 20 ml et la charge d'alumine (Al₂O₃, Harshaw Al 3996 R) de 10,8 g. 48,2 g de 3,3-diméthyl-5-méthyl-5-hexén-2-one préparée selon a) ont été introduits dans un évaporateur de façon à ce que le débit soit de 7 ml/h, avec une coalimentation en eau de 7ml/h.
   Après condensation des vapeurs résultantes, on a obtenu un mélange contenant 80% du cyclopentadiène désiré et quelques produits minoritaires. La purification de ce mélange sur colonne Fischer a fourni le cyclopentadiène désiré pur.
   P. eb. 37°/5,0 x 10⁴ Pa.
   RMN(¹H, 360MHz, CDCl₃) : 1,02(s, 6H) ; 1,78(d, J=2Hz, 2H) ; 1,86(d, J=2Hz, 2H) ; 5,71(s large, 1H) ; 5,75(s large, 1H) δ ppm.
   SM : 122(35, M⁺), 107(160), 91(56), 79(39), 77(14), 65(10).

## Revendications

1. Procédé pour la préparation d'un composé cyclique polyinsaturé de formule 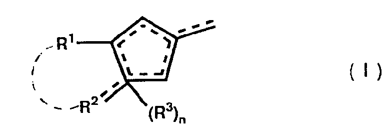 possédant deux doubles liaisons conjugées dans les positions endo- et exo- du cycle pentagonal indiquées par les lignes pointillées, et dans laquelle les symboles R¹ et R², pris séparément, représentent respectivement a. un radical alkyle linéaire ou ramifié de C₁ à C₄ et b. un atome d'hydrogène ou un radical méthyle, ou, pris ensemble avec les atomes de carbone auxquels ils sont liés, forment un cycle polyméthylénique tel qu'indiqué par la ligne pointillée contenant de 5 à 12 atomes de carbone, et le symbole R³ représente un atome d'hydrogène ou un radical méthyle, n étant égal à 0 ou 1, le procédé étant caractérisé en ce qu'on cyclise en phase vapeur une énone de formule 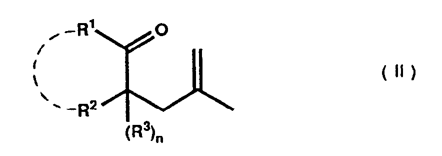 dans laquelle la ligne pointillée et les symboles n, R¹, R², R³ ont le sens indiqué ci-dessus, au moyen d'un traitement thermique en présence d'un catalyseur de cyclisation constitué par un oxyde d'aluminium, de silicium, de titane, de zinc ou de zirconium.

2. Procédé selon la revendication 1, caractérisé en ce qu'on utilise, comme catalyseur de cyclisation, l'alumine.

3. Procédé selon la revendication 1, caractérisé en ce qu'on effectue la cyclisation à une température comprise entre 200 et 400°C.

4. Procédé selon la revendication 3, caractérisé en ce qu'on effectue la cyclisation à une température comprise entre 280 et 320°C.

5. Procédé selon la revendication 1, caractérisé en ce qu'on effectue la cyclisation sous une pression réduite comprise entre 1 x 10³ Pa et 13 x 10³ Pa.

## Patentansprüche

1. Verfahren zur Herstellung von einer cyclischen mehrfach ungesättigten Verbindung der Formel 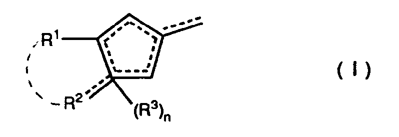 die, wie durch die gestrichelten Linien angezeigt ist, zwei konjugierte Doppelbindungen in den Endo- und Exo-stellungen des Fünfrings enthält, und worin die Symbole R¹ und R², getrennt genommen, entweder a. einen C₁ bis C₄ linearen oder verzweigten Alkylrest und b. ein Wasserstoffatom oder einen Alkylrest bedeuten, oder, zusammen mit den Kohlenstoffatoms an die sie gebunden sind, einen 5 bis 12 Kohlenstoffatoms enhaltender Polymethylenring, bilden, das Symbol R³ ein Wasserstoffatom oder einen Alkylrest darstellt und n für 0 oder 1 steht, dadurch gekennzeichnet, daß man, mittels einer Wärmebehandlung in Gegenwart eines Cyclisierungskatalysators, welcher aus einem Aluminium-, Silicium-, Titan-, Zink- oder Zirconoxyd besteht, ein Enon der Formel, 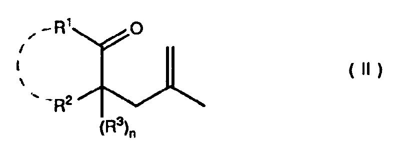 worin die gestrichelte Linie und die Symbole n, R¹, R², R³ die obenangegebene Bedeutung haben, in Dampfphase cyclisiert.

2. Verfahren gemäß Anspruch 1, dadurch gekennzeichnet, daß man als Cyclisierungskatalysator Aluminiumoxyd verwendet.

3. Verfahren gemäß Anspruch 1, dadurch gekennzeichnet, daß die Cyclisierungsreaktion bei einer Temperatur zwischen 200° und 400°C durchgeführt wird.

4. Verfahren gemäß Anspruch 3, dadurch gekennzeichnet, daß die Cyclisierungsreaktion bei einer Temperatur zwischen 280° und 320°C durchgeführt wird.

5. Verfahren gemäß Anspruch 1, dadurch gekennzeichnet, daß die Cyclisierungsreaktion unter vermindertem Druck zwischen 1 x 10³ Pa und 13 x 10³ Pa durchgeführt wird.

## Claims

1. Process for the preparation of a polyunsaturated cyclic compound of formula 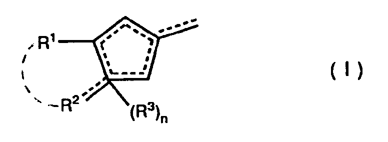 having two conjugated double bonds in the endo- and exo- positions of the pentagonal cycle indicated by the dotted lines, and wherein symbols R¹ and R², when taken separately, represent respectively a. a linear or branched C₁ to C₄ alkyl radical and b. a hydrogen atom or a methyl radical, or, when taken together with the carbon atoms to which they are bonded form a polymethylenic cycle such as indicated by the dotted line, containing from 5 to 12 carbon atoms, and symbol R³ stands for a hydrogen atom or a methyl radical, n being equal to 0 or 1, said process being characterized in that one cyclizes, in the vapour phase, an enone of formula 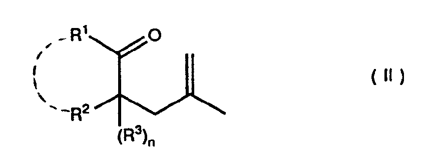 wherein the dotted line and the symbols n, R¹, R² and R³ are defined as above, by means of a thermal treatment in the presence of a cyclization catalyst consisting of an aluminum, silicium, titanium, zinc or zirconium oxide.

2. Process according to claim 1, characterized in that alumina is used as the cyclization catalyst.

3. Process according to claim 1, characterized in that the cyclization is carried out at a temperature comprised between 200° and 400°C.

4. Process according to claim 3, characterized in that the cyclization is carried out at a temperature comprised between 280° and 320°C.

5. Process according to claim 1, characterized in that the cyclization is carried out at a reduced pressure comprised between 1 x 10³ Pa and 13 x 10³ Pa.
